# EUROPEAN PATENT SPECIFICATION

(11) **EP 0 373 151 B1**
(45) Date of publication and mention of the grant of the patent: **15.09.1993**
(21) Application number: 90101204.7
(22) Date of filing: 30.07.1987
(51) Int. Cl.: C07C 227/16

(54) **Process for producing 2-(4,4'-bis-(dimethylamino)-benzhydryl)-5-dimethyl-aminobenzoic acid**
Verfahren zur Produktion von 2-(4,4'-bis(Dimethylamino)-benzhydryl)-5-dimethylaminobenzoesäure
Procédé de production de l'acide 2-(4,4'-bis(diméthylamino)-benzhydryl)-5-diméthylamino benzoique

(30) Priority: 18.05.1987 JP 120934/87
(43) Date of publication of application: 13.06.1990
(62) Divisional of application: 87306758.1
(73) Proprietor: YAMADA CHEMICAL CO., LTD., Kyoto-shi Kyoto-fu (JP)
(72) Inventor: Fujino, Yoshiharu, Tsuzuki-gun, Kyoto-fu (JP); Kawai, Hajime, Tsuzuki-gun, Kyoto-fu (JP); Tsunemitsu, Katushiko, Kyoto-shi, Kyoto-fu (JP)
(74) Representative: Woods, Geoffrey Corlett

(56) References cited:
- FR-A- 2 114 820
- US-A- 2 417 897
- CHEMICAL ABSTRACTS, vol. 85, part 20, 15th November 1976, page 92, abstract no. 144715q, Columbus, Ohio, US; & JP-A-76 48 639
- CHEMICAL ABSTRACTS, vol. 109, part 23, 5th December 1988, page 630, abstract no. 210710b, Columbus, Ohio, US; & JP-A-62 255 454

## Description

The present invention relates to a process for producing 2-[4,4'-bis-(dimethylamino)-benzhydryl]-5-dimethyl-aminobenzoic acid (hereinafter referred to as LC).

LC may be used as an intermediate in the production of 3,3-bis-(4-dimethylaminophenyl)-6-dimethylaminophthalide (crystalviolet lactone, hereinafter referred to as CVL). This use of LC is described and claimed in EP-B-291600, from which this application is divided.

Many other processes of oxidizing LC into CVL are also known. Reference can be made to US-A-2,417,897, JP-A-48-25730 (1973) (US-A-3828071), JP-A-52-78867 (1977), JP-A-52-31384 (1977) and JP-B-59-19548 (1984) (US-A-4271075).

As a process for synthesizing LC as the intermediate for producing CVL, a process of reacting m-dimethylaminobenzoic acid (hereinafter referred to as DABA) with tetramethyl-4,4'-diaminobenzhydrol (hereinafter referred to as MH) in an aqueous solution of sulphuric acid (refer to US-A-3,842,103) and a process of dropping an aqueous sulphuric acid solution of MH into an aqueous sulphuric acid solution of DABA (refer to DE-C-2156648) have been known. However, in each of the two processes, the amount of by-products formed by decomposition is large and the yield and the quality of LC are extremely low.

It has been known that the yield and quality of CVL synthesized by oxidation of LC are generally influenced by the quality of LC, and accordingly isolation and purification of LC are ordinarily carried out before subjecting LC to oxidation. For instance, although there is a method of treating LC in a mixture with an aromatic hydrocarbon or a water-insoluble alkane [refer to JP-A-53-115740 (1978) (CA-A-1087202)], there are the disadvantages that the recovery of the solvent is troublesome and that there is a large loss of LC in the recovery treatment.

Moreover, the process for producing CVL consists essentially of a first step of reacting DABA with MH to form LC and a second step of producing CVL by oxidizing the thus obtained LC. According to the conventional process, LC obtained in the first step is generally separated and purified, and then the separated and purified LC is used as the starting material in the second step. However, since the loss of LC in the isolation and purification thereof is large, a method is also considered wherein the reaction mixture obtained in the first step is used as the starting material in the second step without separating and purifying, and the second step is carried out continuously after the first step.

However, in such a method, not only do the remaining raw material, the decomposition products of the raw material and the by-products of reaction of the first step badly influence the yield of CVL in the second step but also, according to the conditions in the second step, the operations such as the separation of the tarry by-products and the recovery of the solvent accompanying the purification of the CVL are complex.

Particularly, in the first step, large amounts of by-products other than LC are formed, and in consideration of only this point, the process of continuously carrying out the reaction of the first step and the reaction of the second step is inadequate.

The present inventors have found a process for synthesizing LC of an extremely high quality and in a high yield.

The present invention provides a process for producing 2-[4,4'-bis-(dimethylamino)-benzhydryl]-5-dimethylaminobenzoic acid (LC) in the form of particles having a mean particle diameter of not larger than 20 µm, which comprises adding tetramethyl-4,4'-diaminobenzhydrol (MH) in the form of particles having a mean particle diameter of not larger than 50 µm to an aqueous sulphuric acid or hydrochloric acid solution having a pH of from 1.5 to 3.0 containing m-dimethylaminobenzoic acid (DABA) in the form of particles having a mean particle diameter of not larger than 30 µm at a temperature of from 50 to 100°C.

The mean particle diameter of DABA is preferably not larger than 5 µm. The mean particle diameter of MH is preferably not larger than 10 µm.

The reaction of DABA and MH is preferably carried out at a temperature of from 60 to 80°C, and it is advantageous to add the MH as slowly as possible. The addition of a surfactant, for instance polyoxyethylene alkyl ether, to the reaction system is effective for uniformly dispersing the reaction mixture.

The reaction of DABA and MH proceeds under the most suitable reaction conditions at a pH of from 2.0 to 3.0, preferably in a sulphuric acid solution. The reaction is remarkably accelerated under these conditions and only small amounts of by-products are formed.

LC formed in the above-mentioned pH range does not dissolve much in the reaction solution; on the other hand, DABA and MH dissolve well in the reaction solution. Accordingly, MH added thereto rapidly dissolves in the reaction solution and reacts with DABA. Furthermore, DABA suspended in the reaction solution dissolves rapidly with the separation of LC which is formed by the reaction; accordingly the pH of the reaction solution is kept within the above-mentioned range. As a result, LC is formed smoothly and the objective product is obtained in a high yield. Furthermore, since the impurities in DABA and MH and the by-products formed by decomposition dissolve well in the reaction solution in the above-mentioned pH range, such impurities and by-products can be completely removed merely by filtering the thus separated LC after reaction and washing the thus filtered LC with water. Consequently LC of a high quality can be obtained.

The LC is preferably obtained in the form of particles having a mean particle diameter of not larger than 10 µm.

When MH is slowly added to the aqueous sulphuric acid or hydrochloric acid solution containing DABA the MH dissolves in the solution and at the same time the MH reacts with the DABA dissolved in the solution to form LC. Almost all of the thus formed LC separates from the solution except for a small amount.

With the formation and separation of LC, DABA which has been in a non-dissolved state begins to dissolve, and finally all the DABA reacts with MH.

After the addition of MH is finished, and the reaction of DABA and MH is completely finished, the reaction mixture may be filtered, and the thus obtained residue washed with water to neutrality and dried to obtain a dried crude product. As a result, a dried product containing an amount of LC corresponding to 90% of the theoretical amount is obtained.

MH in powdery form or an aqueous dispersion containing a powdery form of MH may be slowly added to the aqueous mineral acid solution containing DABA. The latter method is particularly preferred since the dissolution of MH in the solution containing DABA can be carried out with favourable reproducibility without regard to the purity of MH.

According to the process of the present invention, LC of high quality can be obtained in a high yield.

The present invention is explained in more detail in the following Examples.

### EXAMPLE 1

Into 500ml of an aqueous 4.9% by weight solution of sulphuric acid, 177g of DABA (containing 173g of pure DABA in particles having a mean particle diameter of not larger than 30 µm) were added and after further adding 1g of SCOUROL® #100 (made by KAO-ATLAS CO.) to the thus formed mixture, a mixture of 284g of MH (containing 270g of pure MH in particles having a mean particle diameter of not larger than 50 µm) and 500ml of water was added to the mixture within 10 hours at 70°C under agitation. After the reaction was over, the reaction mixture was subjected to filtration and the residue was washed with water to the neutral point to obtain 391g of the dried crude product (containing an amount of LC corresponding to 92% of the theoretical amount).

### EXAMPLE 2

In the same manner as in Example 1 except for using 310g of MH (containing 270g of pure MH in particles having a mean particle diameter of not larger than 10 µm) instead of the 284g of MH used in Example 1, 387g of the dried crude product (containing an amount of LC corresponding to 90% of the theoretical amount) were obtained.

### EXAMPLE 3

In the same manner as in Example 1 except for adding MH within 5 hours, 390g of the dried crude product (containing an amount of LC corresponding to 90% of the theoretical amount) were obtained.

### EXAMPLE 4

In the same manner as in Example 1 except for using an aqueous 9.8% by weight solution of sulphuric acid instead of the aqueous solution of sulphuric acid in Example 1, 387g of the dried crude product (containing an amount of LC corresponding to 88% of the theoretical amount) were obtained.

## Claims

1. A process for producing 2-[4,4'-bis(dimethylamino)-benzhydryl]-5-dimethyl-aminobenzoic acid (LC) in the form of particles having a mean particle diameter of not larger than 20 µm, which comprises adding tetramethyl-4,4'-diaminobenzhydrol (MH) in the form of particles having a mean particle diameter of not larger than 50 µm to an aqueous sulphuric acid or hydrochloric acid solution having a pH of from 1.5 to 3.0 containing m-dimethylaminobenzoic acid (DABA) in the form of particles having a mean particle diameter of not larger than 30 µm at a temperature of from 50 to 100°C.

2. A process according to claim 1, wherein said MH is added in the form of an aqueous dispersion.

3. A process according to claim 1 or 2, wherein said DABA is in the form of particles having a mean particle diameter of not larger than 5 µm.

4. A process according to any one of the preceding claims, wherein said MH is in the form of particles having a mean particle diameter of not larger than 10 µm.

5. A process according to any one of the preceding claims, wherein the temperature is from 60 to 80°C.

6. A process according to any one of the preceding claims, wherein the pH is from 2.0 to 3.0.

7. A process according to any one of the preceding claims wherein a surfactant is added to the reaction system.

8. A process according to any one of the preceding claims wherein the reaction mixture is filtered and the thus obtained residue is washed with water to neutrality and dried to obtain LC.

## Patentansprüche

1. Verfahren zur Herstellung von 2-[4,4'-Bis-(dimethylamino)-benzhydryl]-5-dimethylaminobenzoesäure (LC) in Form von Partikeln mit einem mittleren Partikeldurchmesser von nicht mehr als 20 µm, umfassend die Zugabe von Tetramethyl-4,4'diaminobenzhydrol (MH) in Form von Partikeln mit einem mittleren Partikeldurchmesser von nicht mehr als 50 µm zu einer wässrigen Schwefelsäure- oder Salzsäurelösung mit einem pH von 1,5 bis 3,0, die m-Dimethylaminobenzoesäure (DABA) in Form von Partikeln mit einem mittleren Partikeldurchmesser von nicht mehr als 30 µm enthält, bei einer Temperatur von 50 bis 100°C.

2. Verfahren gemäss Anspruch 1, dadurch **gekennzeichnet,** dass das MH in Form einer wässrigen Dispeision zugegeben wird.

3. Verfahren gemäss Anspruch 1 oder 2, dadurch **gekennzeichnet,** dass die DABA in Form von Partikeln mit einem mittleren Partikeldurchmesser von nicht mehr als 5 µm zugegeben wird.

4. Verfahren gemäss einem der vorhergehenden Ansprüche, dadurch **gekennzeichnet,** dass MH in Form von Partikeln mit einem mittleren Partikeldurchmesser von nicht mehr als 10 µm vorliegt.

5. Verfahren gemäss einem der vorhergehenden Ansprüche, dadurch **gekennzeichnet,** dass die Temperatur 60 bis 80°C beträgt.

6. Verfahren gemäss einem der vorhergehenden Ansprüche, dadurch **gekennzeichnet,** dass der pH 2,0 bis 3,0 beträgt.

7. Verfahren gemäss einem der vorhergehenden Ansprüche, dadurch **gekennzeichnet,** dass ein Tensid zum Reaktionssystem zugesetzt wird.

8. Verfahren gemäss einem der vorhergehenden Ansprüche, dadurch **gekennzeichnet,** dass die Reaktionsmischung filtriert wird und der so erhaltene Rückstand mit Wasser bis zur Neutralreaktion gewaschen und getrocknet wird, wodurch LC erhalten wird.

## Revendications

1. Procédé de production de l'acide 2-[4,4'-bis(diméthylamino)benzhydryl]-5-diméthylaminobenzoïque (LC) sous forme de particules possédant un diamètre moyen de particules d'au plus 20 µm, procédé qui comprend l'addition de tétraméthyl-4 ,4'-diaminobenzhydrol (MH), sous forme de particules possédant un diamètre moyen de particules d'au plus 50 µm, à une solution aqueuse d'acide sulfurique ou d'acide chlorhydrique ayant un pH de 1,5 à 3,0, contenant de l'acide m-diméthylaminobenzoïque (DABA), sous forme de particules possédant un diamètre moyen de particules d'au plus 30 µm, à une température de 50 à 100°C.

2. Procédé selon la revendication 1, dans lequel ledit MH est ajouté sous la forme d'une dispersion aqueuse.

3. Procédé selon la revendication 1 ou 2, dans lequel ledit DABA se trouve sous la forme de particules possédant un diamètre moyen de particules d'au plus 5 µm.

4. Procédé selon l'une quelconque des revendications précédentes, dans lequel ledit MH se trouve sous la forme de particules possédant un diamètre moyen de particules d'au plus 10 µm.

5. Procédé selon l'une quelconque des revendications précédentes, dans lequel la température est de 60 à 80°C.

6. Procédé selon l'une quelconque des revendications précédentes, dans lequel le pH est de 2,0 à 3,0.

7. Procédé selon l'une quelconque des revendications précédentes, dans lequel un tensioactif est ajouté au milieu réactionnel.

8. Procédé selon l'une quelconque des revendications précédentes, dans lequel le mélange réactionnel est filtré et le résidu ainsi obtenu est lavé à l'eau jusqu'à la neutralité et séché pour donner le LC.
